# EUROPEAN PATENT SPECIFICATION

(11) **EP 3 511 713 B1**
(45) Date of publication and mention of the grant of the patent: **27.10.2021**
(21) Application number: 17848733.6
(22) Date of filing: 05.09.2017
(51) Int. Cl.: G01N 33/574, G01N 33/531, G01N 33/543

(54) **TUMOR MARKER MEASUREMENT METHOD AND MEASUREMENT REAGENT**
TUMORMARKERMESSVERFAHREN UND -MESSREAGENZ
MÉTHODE DE MESURE DE MARQUEUR TUMORAL ET RÉACTIF DE MESURE

(30) Priority: 06.09.2016 JP 2016173878
(43) Date of publication of application: 17.07.2019
(73) Proprietor: Fujirebio Inc., Tokyo 163-0410 (JP)
(72) Inventor: YAMAMOTO, Kosuke, Hachioji-shi Tokyo 192-0031 (JP); KITAMURA, Yoshiyuki, Hachioji-shi Tokyo 192-0031 (JP); YAGI, Shintaro, Hachioji-shi Tokyo 192-0031 (JP); AOYAGI, Katsumi, Hachioji-shi Tokyo 192-0031 (JP)
(74) Representative: Mewburn Ellis LLP
(86) International application number: PCT/JP2017/031874
(87) International publication number: WO 2018/047793

(56) References cited:
- EP-A1- 1 816 460
- EP-A1- 2 708 893
- WO-A1-2015/182580
- JP-A- H08 322 566
- JP-A- 2002 508 963
- JP-A- 2015 531 487

## Description

### TECHNICAL FIELD

The present invention relates to a measurement method and a measurement reagent for a tumor marker.

### BACKGROUND ART

Tumor marker is a general term for proteins which are usually not produced by normal cells, but are specifically produced by tumor cells, which proteins can be detected or measured especially from biological samples such as blood of subjects. In diagnosis of a tumor, a measured value of a tumor marker in a biological sample can be used for judgment of the presence or absence of development of the tumor, judgment of disappearance of the tumor after treatment of a patient, or the like. There are a variety of types of organ-specific tumor markers, and major known examples of the tumor markers include PSA (prostate), α-fetoprotein (AFP, mainly liver), CA125 (ovary), CA15-3 (breast), CA19-9 (digestive organs), and carcinoembryonic antigen (CEA, mainly digestive organs).

Tumor markers in biological samples are often measured by immunoassay systems using antibodies that specifically bind to the tumor markers. However, there are cases where an antibody that binds to a tumor marker, that is, the so-called autoantibody or the like, is present in a biological sample, and it is known that, when such a sample is subjected to the measurement, there is a possibility that the measured value of the tumor marker is lower than its actual value, that is, there is a possibility that a falsely low value is obtained (Non-patent Documents 1 and 2). On the other hand, in treatment of a patient, in cases where the patient received treatment for elimination of cancer cells using a mouse monoclonal antibody that binds to an epitope on the tumor cells, production of an anti-mouse antibody (HAMA) sometimes occur in serum of the patient, and it is known that a measured value of a tumor marker higher than the actual value, that is, a falsely high value, may be obtained due to the influence of the HAMA (Non-patent Document 3). Furthermore, it has been reported that, for an unknown reason, HAMA and anti-animal antibody (HAAA: human anti-animal antibody, which, together with HAMA, is hereinafter referred to as HAMA or the like), and heterophile antibody (which, together with antibody, is hereinafter referred to as autoantibody or the like) observed as low-affinity autoantibody are present in blood, and cause falsely high values and falsely low values (Non-patent Document 4) JP 2015-531487 describes a method of measuring an analyte, such as protein A, after dissociating a complex of the analyte and another moiety, such a complex of Protein A and IgG.

### PRIOR ART DOCUMENTS

### [Non-patent Documents]

[Non-patent Document 1] Miyuki Hyoki et al., JICLA Vol. 37, No. 1, pp. 17-20 (2012)
[Non-patent Document 2] Masaki Abe et al., Kensa to Gijutsu Vol. 40, No. 2, pp. 162-163 (2012)
[Non-patent Document 3] F. J. Primus et al., Clin. Chem. 34/2, pp. 261-264 (1988)
[Non-patent Document 4] Kricka, L. J., Clin. Chem. 45/7, pp. 942-956 (1999)

### SUMMARY OF THE INVENTION

### PROBLEMS TO BE SOLVED BY THE INVENTION

An object of the present invention is to provide a measurement method and use of a measurement reagent in the method that reduce, in measurement of tumor markers in biological samples, occurrence of falsely low values due to the influence of autoantibody or the like, and falsely high values due to the influence of HAMA or the like, thereby enabling more accurate measurement.

### MEANS FOR SOLVING THE PROBLEMS

As a result of intensive study to achieve the above object, the present inventors discovered that, in measurement of a tumor marker in a biological sample, a more accurate measured value of the tumor marker can be obtained without being influenced by autoantibody or the like and HAMA or the like by carrying out, before subjecting the biological sample to an immune reaction, a pretreatment step of mixing the biological sample with a pretreatment liquid containing one or both of a surfactant and an acidifier, thereby completing the present invention. The measurement of the present invention is performed by immunoassay being a direct competitive immunoassay, indirect competitive immunoassay or sandwich immunoassay and the sample being serum or plasma.

The present invention has the following constitution.
(1) A method of measuring, by immunoassay, a tumor marker in a serum or plasma sample separated from a body, the method comprising a pretreatment step of mixing the sample separated from a body with a pretreatment liquid containing an anionic surfactant or an acidifier, wherein the tumor marker in the sample is released from auto-antibody and/or non-specific reactions by human anti-animal antibodies are inhibited, and wherein the immunoassay is a direct competitive immunoassay, indirect competitive immunoassay, or sandwich immunoassay.
(2) The method according to (1), wherein the pretreatment liquid contains an acidifier, and the acidifier has a final concentration of more than 0.05 N and not more than 0.5 N in the pretreatment step.
(3) The method according to (1), wherein the pretreatment liquid contains an anionic surfactant.
(4) The method according to (3), wherein the pretreatment step is carried out under heat.
(5) The method according to any one of (1) to (4), wherein the tumor marker is PSA, α-fetoprotein, CA125, CA15-3, CA19-9, or carcinoembryonic antigen.
(6) Use of a reagent in a method of measuring a tumor marker by immunoassay according to claim 1, the reagent comprising a pretreatment liquid containing an anionic surfactant or an acidifier.

### EFFECT OF THE INVENTION

According to the present invention, a measurement method and a measurement reagent can be provided, which method and reagent can reduce, in measurement of tumor markers in biological samples, occurrence of falsely low values due to the influence of autoantibody or the like, and falsely high values due to the influence of HAMA or the like, thereby enabling more accurate measurement.

### BRIEF DESCRIPTION OF THE DRAWINGS

Fig. 1 is a graph showing correlation between the hydrochloric acid concentration in the acidification pretreatment liquid and the measured values for a serum sample showing a falsely high value of CA19-9.
Fig. 2 is a graph showing correlation between the hydrochloric acid concentration in the acidification pretreatment liquid and the measured values for CA19-9 standard solutions.
Fig. 3 is a graph showing correlation between the hydrochloric acid concentration in the acidification pretreatment liquid and the measured values for a CA19-9 autoantibody model sample.
Fig. 4 is a graph showing correlation between the hydrochloric acid concentration in the acidification pretreatment liquid and the measured values for a PSA autoantibody model sample.

### MODE FOR CARRYING OUT THE INVENTION

Unless otherwise specified, the "%" concentration described in the present description is represented as the weight/volume (w/v) concentration.

### <Measurement Method for Tumor Marker>

The tumor marker to be measured by the method of the present invention is, more specifically, a human-derived tumor marker selected from PSA (prostate), α-fetoprotein (AFP, mainly liver), CA125 (ovary), CA15-3 (breast), CA19-9 (digestive organs), and carcinoembryonic antigen (CEA, mainly digestive organs)

### 1. Pretreatment Step

The method of the present invention is a method in which a tumor marker present in a biological sample is measured using immune reaction by reacting the biological sample with an antibody. The method is characterized in that it includes a pretreatment step of mixing the biological sample with a pretreatment liquid before the immune reaction (reaction step). By the pretreatment step, the tumor marker can be brought into a state in which it is released from autoantibody or the like, and non-specific reactions by HAMA or the like can be inhibited. The pretreatment liquid may contain one of a surfactant and an acidifier, or may contain both of these. The pretreatment liquid preferably contains a surfactant or an acidifier.

The volume ratio between the biological sample and the pretreatment liquid to be mixed in the pretreatment step is preferably 1:10 to 10:1, more preferably 1:5 to 5:1, still more preferably 1:3 to 3:1. The biological sample to be used in the present invention is serum or plasma.

The surfactant to be contained in the pretreatment liquid according to the invention is an anionic surfactant. Preferred examples of the anionic surfactant include sodium dodecyl sulfate (SDS), *N*-lauroyl sarcosine, lithium dodecyl sulfate, sodium dodecylbenzene sulfonate, and deoxycholic acid. SDS may be especially preferably used. In cases where SDS is used, its concentration is preferably 0.1 to 12.5%, more preferably 0.25 to 10%, still more preferably 0.5 to 7.5% in terms of the concentration during the pretreatment of the mixture with the biological sample. In cases where the SDS concentration is 0.1 to 10% (w/v), sufficient release of autoantibody or the like or non-specific substances such as HAMA bound to the tumor marker, and suppression of precipitation and the like of SDS, can be effectively achieved.

Preferred examples of the acidifier contained in the pretreatment liquid include hydrochloric acid, sulfuric acid, and acetic acid. In cases where an acidifier is used, the normality of the acid in the pretreatment liquid, in terms of the concentration during the pretreatment, is preferably more than 0.05N and not more than 0.5 N, especially preferably 0.1N to 0.4N. In cases where the normality of the acid is more than 0.05N and not more than 0.5 N, the effect of the pretreatment can be sufficiently obtained, and influence on the subsequent reaction step can be minimized.

In cases where an acidifier is used for the pretreatment, a cationic surfactant is preferably added in order to prevent occurrence of precipitation upon mixing with the biological sample. The cationic surfactant is especially preferably a cationic surfactant having, in a single molecule, a single-chain alkyl group having 10 or more carbon atoms, and a tertiary amine or a quaternary ammonium salt. Examples of such a surfactant include decyltrimethylammonium chloride, dodecyltrimethylammonium chloride, tetradecyltrimethylammonium chloride, hexadecyltrimethylammonium chloride (C16TAC), decyltrimethylammonium bromide, dodecyltrimethylammonium bromide, tetradecyltrimethylammonium bromide, hexadecyltrimethylammonium bromide (CTAB), laurylpyridinium chloride, tetradecylpyridinium chloride, and cetylpyridinium chloride. The amount of the cationic surfactant to be added, in terms of the concentration after mixing with the sample, is preferably 0.01% to 15%, more preferably 0.05% to 10%.

The pretreatment liquid containing an acidifier may contain, in addition to the above-mentioned cationic surfactant, another surfactant such as a nonionic surfactant. By the addition of the other surfactant, detection of the tumor marker is possible with an even higher sensitivity.

A reducing agent may also be used for the pretreatment liquid. As the reducing agent, any of known reducing agents such as 2-(diethylamino)ethanethiol hydrochloride (DEAET), tris(2-carboxyethyl)phosphine hydrochloride (TCEP), dithiothreitol (DTT), 2-mercaptoethanol, thioglycerol, sodium sulfite, and borohydride may be used. From the viewpoint of stability in the solution, DEAET or TCEP may be especially preferably used. The concentration of the reducing agent, in terms of the final concentration in the mixed liquid with the biological sample, is preferably 0.5 to 100 mM, more preferably 1.0 to 50 mM, still more preferably 2.0 to 20 mM.

When necessary, the pretreatment liquid may contain another protein denaturant such as urea or thiourea. The concentration of the denaturant, in terms of the concentration during the treatment, is preferably not less than 0.1 M, more preferably not less than 0.5 M and less than 4 M. For enhancement of the effect of the treatment, the pretreatment liquid may contain any of monosaccharides, disaccharides, citric acid, and citric acid salts, or a combination of these. The pretreatment liquid may also contain a chelating agent such as EDTA.

In the pretreatment step, the mixing of the biological sample with the pretreatment liquid is preferably further followed by heating. In particular, in cases where a surfactant is used for the pretreatment liquid, heating is preferably carried out in order to increase its effect. The heating temperature is preferably 35 to 95°C, more preferably 50 to 90°C, still more preferably 70 to 85°C. The heating time is preferably not less than 1 minute, more preferably not less than 3 minutes, still more preferably not less than 5 minutes. Although there is no upper limit of the heating time, the heating time may be usually not more than 60 minutes, especially not more than 30 minutes.

### 2. Reaction Step

The biological-sample-mixed liquid treated by the above-described pretreatment step in the method of the present invention is subsequently subjected to the reaction step. In the reaction step, the biological-sample-mixed liquid is mixed with a buffer, and antigen in the mixed liquid is allowed to react with an antibody against the tumor marker to be measured.

Examples of the buffer include those based on MES buffer, phosphate buffer, Tris buffer, and carbonate buffer. Buffers based on phosphate buffer may be especially preferably used. In cases where a pretreatment liquid containing a surfactant is used, for example, a buffer containing a water-soluble polymer such as BSA, polyvinyl pyrrolidone (PVP), polyvinyl alcohol (PVA), or dextran sulfate sodium at about 0.01 to 10%, especially 0.05 to 5.0% in terms of the final concentration after mixing with the pretreated mixed liquid is preferably used. In cases where a pretreatment liquid containing an acidifier is used, it is preferred to use a buffer containing an alkaline agent or having a buffer capacity capable of decreasing the influence of the acid in the pretreatment liquid. The mixed liquid of the pretreatment step and the buffer are mixed at a volume ratio of preferably 1:10 to 10:1, more preferably 1:5 to 5:1, still more preferably 1:3 to 3:1.

The antibody against the tumor marker to be used in the method of the present invention is an antibody that recognizes at least part of the amino acid sequence, or a sugar chain, of the tumor marker as an epitope. The antibody against the tumor marker is not limited, and any antibody that recognizes a known epitope may be used. The antibody against the tumor marker is preferably an antibody that recognizes a tumor marker-specific epitope.

The antibody against the tumor marker may be either a polyclonal antibody or a monoclonal antibody. The antibody against the tumor marker may be any isotype of immunoglobulins (for example, IgG, IgM, IgA, IgD, IgE, or IgY). The antibody against the tumor marker may be a full-length antibody. The full-length antibody means an antibody containing a heavy chain and a light chain each having a variable region and a constant region (for example, an antibody containing two Fab regions and an Fc region). The antibody against the tumor marker may also be an antibody fragment derived from such a full-length antibody. The antibody fragment is part of a full-length antibody, and examples of the antibody fragment include antibodies lacking the constant region (for example, F(ab')2, Fab', Fab, or Fv). The antibody against the tumor marker may also be a modified antibody such as a single-chain antibody.

The antibody against the tumor marker can be prepared using a conventionally known method. For example, the antibody against the tumor marker can be prepared using the above-described epitope as an antigen. Alternatively, since a number of antibodies against tumor markers that recognize the above-described epitopes are commercially available, such commercially available products may also be used.

The antibody against the tumor marker may be immobilized on a solid phase. In the present description, an antibody immobilized on a solid phase may be simply referred to as an immobilized antibody. Examples of the solid phase include solid phases in which a liquid phase can be stored or loaded (for example, supports such as plates, membranes, and test tubes; and containers such as well plates, microchannels, glass capillaries, nanopillars, and monolith columns) and solid phases that can be suspended or dispersed in a liquid phase (for example, solid-phase carriers such as particles). Examples of the material of the solid phase include glasses, plastics, metals, and carbons. As the material of the solid phase, a non-magnetic material or a magnetic material may be used. From the viewpoint of simplicity of operation and the like, the material is preferably a magnetic material. The solid phase is preferably a solid-phase carrier, more preferably a magnetic solid-phase carrier, still more preferably a magnetic particle. As the method for immobilization of the antibody, a conventionally known method may be used. Examples of such a method include physical adsorption, covalent bonding, use of an affinity substance (biotin, streptavidin, or the like), and ionic bonding. In a particular embodiment, the antibody against the tumor marker is an antibody immobilized on a solid phase, preferably an antibody immobilized on a magnetic solid phase, more preferably an antibody immobilized on a magnetic particle.

In the reaction step, after the mixing of the mixed liquid of the pretreatment step with the buffer, the resulting mixture may be brought into contact with the immobilized antibody, or, for example, an antibody immobilized on particles may be preliminarily included in a buffer to provide a particle liquid followed by mixing the above mixed liquid with the particle liquid. Although the reaction step may be carried out by a primary reaction step alone as in the immunoagglutination method or the competitive method, a secondary reaction step may also be provided. In cases where the secondary reaction step is provided, a washing step for removal of unreacted components may be provided between the primary reaction step and the secondary reaction step.

The antibody against the tumor marker may be labeled with a labeling substance. In the present description, an antibody labeled with a labeling substance may be simply referred to as a labeled antibody. Examples of the labeling substance include enzymes (peroxidase, alkaline phosphatase, luciferase, β-galactosidase, and the like), affinity substances (streptavidin, biotin, and the like), fluorescent substances and proteins (fluorescein, fluorescein isothiocyanate, rhodamine, green fluorescent protein, red fluorescent protein, and the like), luminescent or light-absorbing substances (luciferin, aequorin, acridinium, ruthenium, and the like), and radioactive substances (³H, ¹⁴C, ³²P, ³⁵S, ¹²⁵I, and the like). In cases where the secondary reaction is provided in the method of the present invention, the antibody to be used for the secondary reaction may be labeled with such a labeling substance.

In a particular embodiment, the antibody to be used for the secondary reaction in the method of the present invention includes another antibody against the tumor marker that recognizes an epitope different from that of the above antibody against the tumor marker. Details of such an epitope recognized by the other antibody are the same as the details of the epitope of the above-described antibody against the tumor marker (however, in the case of combined use, the types of the epitopes are different). The combination of the epitope recognized by the antibody against the tumor marker and the epitope recognized by the other antibody against the tumor marker is not limited. Use of such another antibody is preferred in cases where, for example, the sandwich method is used.

### 3. Detection Step

In cases where a label is used for the primary antibody or the secondary antibody, the detection is carried out by a method suitable for the label used. For example, in cases where an enzyme label is used, the detection is carried out by adding a substrate of the enzyme. For example, in cases where alkaline phosphatase (ALP) is used for the labeled antibody, 3-(2'-spiroadamantane)-4-methoxy-4-(3'-phosphoryloxy)phenyl-1,2-dioxetane disodium salt (AMPPD) may be used as the enzyme substrate to provide a system of the chemiluminescent enzyme immunoassay (CLEIA) method.

The method of the present invention is an immunoassay using an antibody against a tumor marker. According to the invention the immunoassay is selected from the direct competitive method, indirect competitive method, and sandwich method. Further examples of such an immunoassay as decribed herein include chemiluminescent enzyme immunoassay (CLEIA), chemiluminescence immunoassay (CLIA), turbidimetric immunoassay (TIA), enzyme immunoassay (EIA) (for example, direct competitive ELISA, indirect competitive ELISA, and sandwich ELISA), radioimmunoassay (RIA), latex agglutination, fluoroimmunoassay (FIA), and immunochromatography. These immunoassays *per se* are well known, and do not need to be described herein in detail. A brief description of each immunoassay is given below.

The direct competitive method is a method in which an antibody against a target antigen to be measured (in the present invention, a tumor marker) is immobilized on a solid phase (the solid phase and the immobilization are as described above), and blocking treatment (treatment of the solid phase with a solution of protein such as serum albumin) for prevention of non-specific adsorption is carried out, followed by reacting this antibody with a test sample containing the target antigen (in the present invention, a biological sample subjected to the pretreatment step as described above) and a certain amount of labeled antigen (the label is as described above), performing washing, and then quantifying the label bound to the solid phase. Since the antigen in the test sample and the labeled antigen competitively bind to the antibody, the larger the amount of the antigen in the test sample, the smaller the amount of the label bound to the solid phase. Antigen standard solutions with various known concentrations are prepared, and the amount of the label (the absorbance, luminescence intensity, fluorescence intensity, or the like depending on the properties of the label; the same applies hereinafter) immobilized on the solid phase is measured for each solution, followed by preparation of a calibration curve in which the antigen concentration is taken along the abscissa, and the amount of the label is taken along the ordinate. By measuring the amount of the label for an unknown test sample, and applying the measured amount of the label to the calibration curve, the amount of the antigen in the unknown test sample can be measured. The direct competitive method *per se* is well known in the art, and described in, for example, US 20150166678 A1.

In the indirect competitive method, a target antigen (in the present invention, a tumor marker) is immobilized on a solid phase (the solid phase and the immobilization are as described above). Subsequently, blocking treatment of the solid phase is carried out, and then a test sample containing the target antigen (in the present invention, a biological sample subjected to the pretreatment step as described above) is mixed with a certain amount of an anti-target-antigen antibody, followed by reaction with the immobilized antigen. After washing, the anti-target-antigen antibody bound to the solid phase is quantified. This can be carried out by allowing reaction with a labeled secondary antibody (the label is as described above) against the anti-target-antigen antibody, performing washing, and then measuring the amount of the label. Antigen standard solutions with various known concentrations are prepared, and the amount of the label immobilized on the solid phase is measured for each solution, followed by preparation of a calibration curve. By measuring the amount of the label for an unknown test sample, and applying the measured amount of the label to the calibration curve, the amount of the antigen in the unknown test sample can be measured. It is also possible to use a labeled primary antibody without using the labeled secondary antibody. The indirect competitive method *per se* is well known in the art, and described in, for example, the above-mentioned US 20150166678 A1.

The sandwich method is a method in which an anti-target-antigen antibody is immobilized on a solid phase (the solid phase and the immobilization are as described above), and blocking treatment is carried out, followed by reaction with a test sample containing a target antigen (in the present invention, a biological sample subjected to the pretreatment step as described above), washing, reaction with a labeled secondary antibody against the target antigen (the label is as described above), washing, and then quantification of the label bound to the solid phase. Antigen standard solutions with various known concentrations are prepared, and the amount of the label immobilized on the solid phase is measured for each solution, followed by preparation of a calibration curve. By measuring the amount of the label for an unknown test sample, and applying the measured amount of the label to the calibration curve, the amount of the antigen in the unknown test sample can be measured. The sandwich method *per se* is well known in the art, and described in, for example, US 20150309016 A1.

Among the immunoassays described above, chemiluminescent enzyme immunoassay (CLEIA), chemiluminescence immunoassay (CLIA), enzyme immunoassay (EIA), radioimmunoassay (RIA), and fluoroimmunoassay (FIA) are immunoassays classified based on the type of the label to be used when the direct competitive method, indirect competitive method, or sandwich method described above is carried out. Chemiluminescent enzyme immunoassay (CLEIA) is an immunoassay which uses an enzyme (for example, the above-described alkaline phosphatase) as a label, and uses a substrate that generates a chemiluminescent compound (for example, the above-described AMPPD) as a substrate. Enzyme immunoassay (EIA) is an immunoassay which uses an enzyme (for example, the above-described peroxidase, alkaline phosphatase, luciferase, or β-galactosidase) as a label. As the substrate of each enzyme, a compound quantifiable by measurement of the absorbance or the like is used. For example, in cases of peroxidase, 1,2-phenylenediamine (OPD), 3,3'5,5'-tetramethylbenzidine (TMB), or the like is used. In cases of alkaline phosphatase, *p*-nitrophenyl phosphate (pNPP) or the like is used. In cases of β-galactosidase, MG: 4-methylumbelliferyl galactoside, NG: nitrophenyl galactoside, or the like is used. In cases of luciferase, luciferin or the like is used. Radioimmunoassay (RIA) is a method which uses a radioactive substance as a label. Examples of the radioactive substance include radioactive elements such as ³H, ¹⁴C, ³²P, ³⁵S, and ¹²⁵I as described above. Fluoroimmunoassay (FIA) is a method which uses a fluorescent substance or a fluorescent protein as a label. Examples of the fluorescent substance or the fluorescent protein include, as described above, fluorescein, fluorescein isothiocyanate, rhodamine, green fluorescent protein, and red fluorescent protein. Immunoassays *per se* using these labels are well known in the art, and described in, for example, US 8039223 B and US 20150309016 A1.

Turbidimetric immunoassay (TIA) ,as described herein, is an immunoassay which utilizes the phenomenon that an antigen-antibody complex produced by antigen-antibody reaction between a target antigen to be measured (in the present invention, a tumor marker) and an antibody against this antigen causes an increase in the turbidity. The antigen is added, at various known concentrations, to an anti-target-antigen antibody solution, and the turbidity of each resulting mixture is measured to prepare a calibration curve. By similarly measuring the turbidity of an unknown test sample, and applying the measured turbidity to the calibration curve, the amount of the antigen in the unknown test sample can be measured. Turbidimetric immunoassay *per se* is well known in the art, and described in, for example, US 20140186238 A1. Latex agglutination , as described herein, is a method similar to turbidimetric immunoassay, but uses a suspension of latex particles whose surfaces have an anti-target-antigen antibody immobilized thereon, instead of the antibody solution in turbidimetric immunoassay. Turbidimetric immunoassay and latex agglutination *per se* are well known in the art, and described in, for example, US 820,398 B.

Immunochromatography is a method in which the above-described sandwich method or competitive method is carried out on a substrate (also called a matrix or a strip) formed with a porous material such as filter paper, cellulose membrane, glass fiber, or non-woven fabric. For example, in cases of immunochromatography by the sandwich method, a detection zone on which an anti-target-antigen antibody is immobilized is provided on the substrate, and a test sample containing a target antigen (in the present invention, a biological sample subjected to the pretreatment step as described above) is added to the substrate, followed by allowing a developer to flow from the upstream side, thereby allowing the target antigen to migrate to the detection zone and immobilizing the target antigen on the detection zone. The immobilized target antigen is sandwiched with a labeled secondary antibody, and the label immobilized on the detection zone is detected to detect the target antigen in the test sample. By forming a labeling zone containing the labeled secondary antibody in the upstream side of the detection zone, the conjugate of the target antigen and the labeled secondary antibody is immobilized on the detection zone. In cases where the label is an enzyme, a substrate zone containing a substrate of the enzyme is also provided in the upstream side of the detection zone. In cases of the competitive method, for example, the target antigen may be immobilized on the detection zone, and the target antigen in the test sample may be allowed to compete with the target antigen immobilized on the detection zone. By providing a labeled antibody zone in the upstream side of the detection zone, allowing the target antigen in the test sample to react with the labeled antibody, immobilizing unreacted labeled antibody on the detection zone, and then detecting or quantifying the label, the target antigen in the test sample can be detected or quantified. Immunochromatography *per se* is well known in the art, and described in, for example, US 6210898 B.

### <Measurement Reagent for Tumor Marker>

The measurement reagent for a tumor marker used in the method of the present invention is a measurement reagent that can realize the above-described measurement method for a tumor marker. The measurement reagent used in the method of the present invention is characterized in that it contains, as a constituting component, a pretreatment liquid containing an anionic surfactant or an acidifier, in addition to the constitution used for ordinary immunoassays.

The reagent used in the method of the present invention contains the constituting components in a form in which they are isolated from each other, or in the form of a composition. More specifically, the constituting components may be provided in a form in which they are stored in different containers (for example, tubes or plates), or some of the constituting components may be provided in the form of a composition (for example, in a single solution). Alternatively, the reagent used in the method of the present invention may be provided in the form of a device. More specifically, the reagent may be provided in a form in which all constituting components are stored in a device. Alternatively, the reagent may be provided in a form in which some of the constituting components are stored in a device while the remaining constituting components are not stored in the device (for example, in a form in which they are stored in a different container(s)). In such a case, the constituting components not stored in the device may be used by injection into the device when conducting the measurement of the target substance.

In a preferred embodiment, the reagent used in the method of the present invention may have a constitution suitable for the type of the immunoassay to be employed. For example, in cases where the sandwich method is employed, the reagent of the present invention may contain, as indispensable constituting components, i) a pretreatment liquid, ii) an antibody against a tumor marker, and iii) a buffer; and, as arbitrary constituting components, iv) another antibody against the tumor marker, v) a labeling substance, vi) a diluent, and, when necessary, vii) a substrate that reacts with the labeling substance. The constituting components ii) and iii) may be contained in a single solution. The constituting component iv) may be labeled with the labeling substance v). The antibody against the tumor marker may be preferably immobilized on magnetic particles.

### EXAMPLES

### <Example 1 Avoidance of Interference of Autoantibody by Acidification Pretreatment>

### (1) Sample Preparation

Instead of a sample containing autoantibody or the like, a high-concentration standard solution of Lumipulse Presto CA19-9, AFP, CA15-3, CA125, or CEA 5-fold diluted with a sample dilution liquid (composition: containing Tris buffer and BSA), to which the same antibody, in the free form, as the immobilized antibody of each reagent (derived from mouse) was added at a final concentration of 100 µg/mL, was used as an autoantibody model sample. The concentrations of the standard solutions used are shown in Table 1. In addition, a sample prepared by addition of mouse IgG instead of the immobilized antibody at 100 µg/mL was used as a control sample.

**[Table 1]**

| Tumor marker | High-concentration standard solution |
|---|---|
| CA19-9 | 500 U/mL |
| AFP | 2000 ng/mL |
| CA15-3 | 400 U/mL |
| CA125 | 1000 U/mL |
| CEA | 200 ng/mL |

With 50 µL of an acidification pretreatment liquid (2.5 M urea, 0.42 M hydrochloric acid, 0.08 M citric acid hydrate, 2.5% maltose, 10.0% CTAB, 4.9% Triton X-100 (trade name)), 50 µL of each of the autoantibody model samples and the control samples was mixed, and the resulting mixture was warmed with shaking at 1000 rpm at 37°C for 6 minutes. Subsequently, 100 µL of a buffer (500 mM bicine, 50 mM MOPS, 200 mM NaCl, 20 mM EDTA 3Na, 10.0% BSA, 0.10% ProClin (registered trademark), NaOH (about pH 9.2)) was added thereto to prepare an acidification-pretreated sample. In addition, 50 µL of each of the same samples was mixed with a liquid preliminarily prepared by mixing 50 µL of the acidification pretreatment liquid with 100 µL of the buffer, to provide an untreated sample. For the autoantibody model samples and the control samples, each tumor marker was measured in a normal manner using Lumipulse Presto CA19-9, AFP, CA15-3, CA125, or CEA (Fujirebio Inc.).

The measurement results are shown in Table 2. In the untreated cases, all of the tumor markers CA19-9, AFP, CA15-3, CA125, and CEA underwent inhibition of the reaction by the addition of the immobilized antibody, resulting in remarkably low measured values (count values) relative to the control samples. On the other hand, the samples subjected to the acidification pretreatment avoided the reaction inhibition by the immobilized antibody, resulting in measured values equivalent to those of the control samples. From these results, it was suggested that falsely low values due to autoantibody or the like in a measurement system for a tumor marker can be avoided by acidification pretreatment of the sample.

**[Table 2]**

| | CA19-9 | | AFP | | CA15-3 | | CA125 | | CEA | |
|---|---|---|---|---|---|---|---|---|---|---|
| | Untreated | Pretreated | Untreated | Pretreated | Untreated | Pretreated | Untreated | Pretreated | Untreated | Pretreated |
| Mouse IgG-added standard solution | 96,144 | 92,387 | 481,045 | 238,523 | 450,456 | 435,063 | 38,141 | 43,740 | 91,048 | 32,707 |
| Immobilized antibody-added standard solution | 2,297 | 83,965 | 20,602 | 234,463 | 133,021 | 439,885 | 2,025 | 43,249 | 1,903 | 30,716 |
| Immobilized antibody/ mouse IgG | 2% | 91% | 4% | 98% | 30% | 101% | 5% | 99% | 2% | 94% |

| | | | | | | | | | | |
|---|---|---|---|---|---|---|---|---|---|---|
| (Unit: Counts) | | | | | | | | | | |

### <Example 2 Effect of Acidification Pretreatment on Inhibition of HAMA>

### (1) Sample Pretreatment

Twenty microliters of one sample showing a falsely high value in measurement of CA19-9 due to the influence of HAMA was mixed with 20 µL of various concentrations (0.025 N, 0.05 N, 0.1 N, 0.2 N, 0.4 N, 0.6 N, 0.8 N, 1.0 N, 1.2 N, 1.4 N, 1.6 N, 1.8 N, or 2.0 N) of hydrochloric acid, and each resulting mixture was incubated at 37°C for 10 minutes. Each sample solution was neutralized by adding 40 µL of a solution of sodium hydroxide equimolar to the hydrochloric acid, and then 80 µL of a buffer (24 mM potassium dihydrogen phosphate, 76 mM dipotassium hydrogen phosphate, 1.0% BSA, 1.0% PVP, 0.05% casein sodium, 0.05% Tween 20 (trade name), 0.05% sodium chloride, 20 mM EDTA 2Na, 0.1% Proclin (registered trademark) 300; pH 7.0) was added thereto to provide a measurement sample. In addition, each of the 500 IU/mL and 0 IU/mL standard solutions of Lumipulse CA19-9 was diluted 3-fold with PBS, and subjected to the acidification treatment, neutralization, and addition of the buffer similarly to the sample, to provide a control sample.

Each measurement sample was measured in a normal manner using Lumipulse Presto CA19-9 (Fujirebio Inc.).

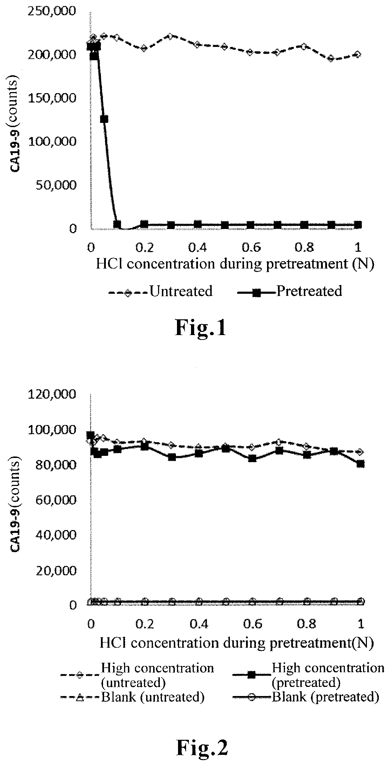

The relationship between the hydrochloric acid concentration during the pretreatment and the count value of the measurement sample (which represents the reaction intensity) is shown in Fig. 1, and the count values of the control samples are shown in Fig. 2. Since the control samples hardly showed changes in the count values due to the hydrochloric acid treatment, it was found that CA19-9 itself was not influenced by the pretreatment. On the other hand, the sample showing a falsely high value due to HAMA showed remarkable decreases in the count value due to the pretreatment with hydrochloric acid especially at the concentrations of not less than 0.1 N. From these results, it was found that the influence of HAMA in measurement of CA19-9 can be avoided by the acidification pretreatment.

### <Example 3 Optimum Concentration for Avoidance of Interference of Autoantibody by Acidification Pretreatment>

The optimum concentration of the acidifier to be used for the acidification pretreatment, which concentration is effective for avoidance of interference of autoantibody, was studied. Each of high-concentration standard solutions of Lumipulse CA19-9 and PSA (CA19-9: 500 IU/mL, PSA: 100 ng/mL) was diluted to 1/10 with negative human serum, and the same antibody, in the free form, as the immobilized antibody of Lumipulse CA19-9 or PSA was added thereto at 100 µg/mL, followed by leaving the resulting mixture to stand at room temperature for 2 hours, to prepare an autoantibody model sample. In addition, a control sample was prepared by adding mouse IgG instead of the immobilized antibody at 100 µg/mL. With 50 µL of an acidification pretreatment liquid (2.5 M urea, hydrochloric acid, 2.5% maltose, 10.0% CTAB, 4.9% Triton X-100), 50 µL of each sample was mixed. The preparation of the acidification pretreatment agent was carried out such that it contained 2 N, 1 N, 0.8 N, 0.6 N, 0.4 N, 0.2 N, 0.1 N, 0.05 N, 0.025 N, or 0 N hydrochloric acid, and the same study was carried out at each concentration. The sample mixed with the acidification pretreatment liquid was warmed with shaking at 1000 rpm at 37°C for 6 minutes. Subsequently, 100 µL of a solution of sodium hydroxide equivalent to the hydrochloric acid was added thereto for neutralization. To the neutralized solution, 200 µL of the same buffer as in Example 2 was added to provide an acidification-pretreated sample. Each acidification-pretreated sample was subjected to measurement in a normal manner using Lumipulse Presto CA19-9 or PSA (Fujirebio Inc.).

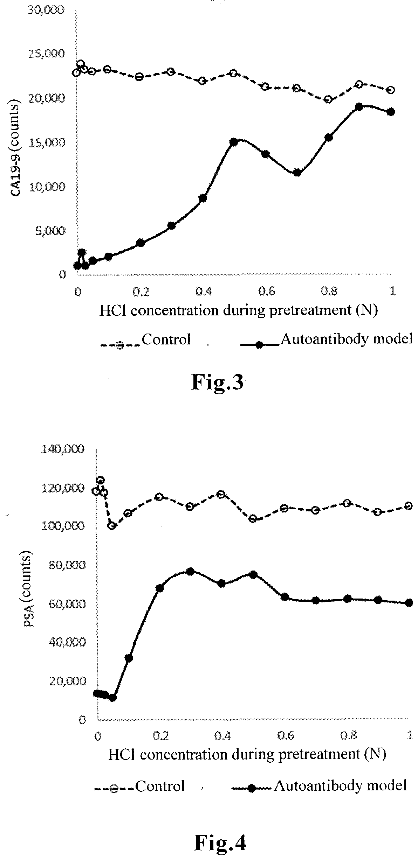

The measurement results for the acidification-pretreated samples of the CA19-9 and PSA autoantibody model samples, as well as their control samples, are shown in Figs. 3 and 4, respectively. The CA19-9 autoantibody model sample tended to show decreased differences, from the control sample, in the measured count value when the hydrochloric acid concentration during the pretreatment was 0.5 N to 0.9 N. On the other hand, the PSA autoantibody model sample tended to show decreased differences, from the control sample, in the count value when the hydrochloric acid concentration during the pretreatment was not less than 0.2N. From these results, it was suggested that occurrence of a false negative due to autoantibody or the like of a tumor marker can be reduced by the acidification pretreatment of the sample. Since these studies were carried out for the model samples using the antibodies having strong affinities to CA19-9 or PSA instead of autoantibody, there is a possibility that occurrence of a falsely low value in an actual autoantibody-containing sample can be reduced even when the acid concentration in the acidification pretreatment agent is lower.

### <Example 4 Effect of SDS Pretreatment on Inhibition of HAMA>

### (1) SDS Pretreatment of Samples

Fifty microliters of each of five samples showing falsely high values in measurement of CA19-9 due to the influence of HAMA was mixed with 100 µL of an SDS pretreatment liquid (10% SDS, 1.5% C14APS, 0.225% Triton X-100 (trade name), 3.75% EDTA 2Na, 37.5 mM L-tryptophan, 150 mM imidazole, 10 mM TCEP), and each resulting mixture was heated with shaking at 1000 rpm at 80°C for 5 minutes. In addition, 100 µL of PBS instead of the pretreatment liquid was added to the same samples to provide untreated samples. Also for the Lumipulse CA19-9 standard solution, an SDS-pretreated sample and an untreated sample were prepared in the same manner.

### (2) Measurement of CA19-9

Measurement of each sample was carried out using Lumipulse Presto CA19-9 (Fujirebio Inc.), which is a commercially available measurement reagent for CA19-9, and an autoanalyzer Lumipulse Presto II (Fujirebio Inc.). The measurement reagent was used in the same manner as the ordinary method except that a mixture of the magnetic particle liquid and 20% BSA solution at a volume ratio of 5:1 was used instead of the normal magnetic particle liquid.

### (3) Results

Table 3 shows comparison of the CA19-9 count value between the SDS-pretreated samples and the untreated samples. All samples showed remarkable decreases in the count value. On the other hand, since the count value for the standard solution did not show a large change, it was suggested that falsely high values of HAMA can specifically be avoided by the pretreatment, without negatively influencing the CA19-9 molecule itself.

**[Table 3]**

| Sample No. | Measured value (counts) | | |
|---|---|---|---|
| | Untreated | Pretreated | Pretreated/ Untreated |
| 1 | 113,087 | 33,783 | 30% |
| 2 | 992,619 | 11,518 | 1% |
| 3 | 849,855 | 10,031 | 1% |
| 4 | 840,282 | 7,015 | 1% |
| 5 | 528,059 | 42,299 | 8% |

### <Example 5 Avoidance of Interference of Autoantibody by SDS Pretreatment>

An autoantibody model sample of CA19-9 and its control sample were prepared by the same method as in Example 1, and sample pretreatment and measurement of CA19-9 were carried out by the same method as in Example 4. The measurement results for CA19-9 are shown in Table 4. In the untreated case, the autoantibody model sample showed inhibition of the reaction due to the addition of the immobilized antibody, resulting in a remarkably low count value relative to the control sample. On the other hand, the sample subjected to the SDS pretreatment avoided the reaction inhibition by the immobilized antibody, resulting in a measured value equivalent to that of the control sample. Thus, it was suggested that falsely low values due to autoantibody or the like in a measurement system for a tumor marker can be avoided by SDS pretreatment of the sample.

**[Table 4]**

| | CA19-9 (counts) | |
|---|---|---|
| | Untreated | Pretreated |
| Mouse IgG-added standard solution | 118,150 | 110,974 |
| Immobilized antibody-added standard solution | 2,174 | 110,057 |
| Immobilized antibody/ mouse IgG | 2% | 99% |

## Claims

1. A method of measuring, by immunoassay, a tumor marker in a serum or plasma sample separated from a body, the method comprising a pretreatment step of mixing the sample separated from a body with a pretreatment liquid containing an anionic surfactant or an acidifier, wherein the tumor marker in the sample is released from auto-antibody and/or non-specific reactions by human anti-animal antibodies are inhibited, and wherein the immunoassay is a direct competitive immunoassay, indirect competitive immunoassay, or sandwich immunoassay.

2. The method according to claim 1, wherein the pretreatment liquid contains an acidifier, and the acidifier has a final concentration of more than 0.05 N and not more than 0.5 N in the pretreatment step.

3. The method according to claim 1, wherein the pretreatment liquid contains an anionic surfactant.

4. The method according to claim 3, wherein the pretreatment step is carried out under heat.

5. The method according to any one of claims 1 to 4, wherein the tumor marker is PSA, α-fetoprotein, CA125, CA15-3, CA19-9, or carcinoembryonic antigen.

6. Use of a reagent in a method of measuring a tumor marker by immunoassay, according to claim 1, the reagent comprising a pretreatment liquid containing an anionic surfactant or an acidifier.

## Patentansprüche

1. Verfahren zum Messen eines Tumormarkers in einer Serum- oder Plasmaprobe, die aus einem Körper erhalten wurde, durch einen Immunassay, wobei das Verfahren einen Vorbehandlungsschritt des Vermischens der aus dem Körper erhaltenen Probe mit einer Vorbehandlungsflüssigkeit, die ein anionisches Tensid oder einen Säurebildner enthält, umfasst, wobei der Tumormarker in der Probe aus Auto-Antikörper- und/oder nichtspezifischen Reaktionen durch menschliche Anti-Tier-Antikörper freigesetzt wird und wobei der Immunassay ein direkter kompetitiver Immunassay, in indirekter kompetitiver Immunassay oder ein Sandwich-Immunassay ist.

2. Verfahren nach Anspruch 1, wobei die Vorbehandlungsflüssigkeit einen Säurebildner enthält und wobei der Säurebildner eine Endkonzentration von mehr als 0,05 N und nicht mehr als 0,5 N in dem Vorbehandlungsschritt aufweist.

3. Verfahren nach Anspruch 1, wobei die Vorbehandlungsflüssigkeit ein anionisches Tensid enthält.

4. Verfahren nach Anspruch 3, wobei der Vorbehandlungsschritt unter Erwärmen durchgeführt wird.

5. Verfahren nach einem der Ansprüche 1 bis 4, wobei der Tumormarker PSA, α-Fetoprotein, CA125, CA15-3, CA19-9 oder karzinoembryonales Antigen ist.

6. Verwendung eines Reagens in einem Verfahren zum Messen eines Tumormarkers durch einen Immunassay nach Anspruch 1, wobei das Reagens eine Vorbehandlungsflüssigkeit, enthaltend ein anionisches Tensid oder einen Säurebildner, umfasst.

## Revendications

1. Procédé de mesure, par dosage immunologique, d'un marqueur tumoral dans un échantillon de sérum ou de plasma séparé d'un corps, le procédé comprenant une étape de prétraitement consistant à mélanger l'échantillon séparé d'un corps avec un liquide de prétraitement contenant un tensioactif anionique ou un acidifiant, dans lequel le marqueur tumoral dans l'échantillon est libéré d'un auto-anticorps et/ou des réactions non-spécifiques par des anticorps humains anti-animal sont inhibées, et dans lequel le dosage immunologique est un dosage immunologique compétitif direct, un dosage immunologique compétitif indirect, ou un dosage immunologique de type sandwich.

2. Procédé selon la revendication 1, dans lequel le liquide de prétraitement contient un acidifiant, et l'acidifiant a une concentration finale de plus de 0,05 N et de pas plus de 0,5 N dans l'étape de prétraitement.

3. Procédé selon la revendication 1, dans lequel le liquide de prétraitement contient un tensioactif anionique.

4. Procédé selon la revendication 3, dans lequel l'étape de prétraitement est effectuée sous chaleur.

5. Procédé selon l'une quelconque des revendications 1 à 4, dans lequel le marqueur tumoral est PSA, α-foetoprotéine, CA125, CA15-3, CA19-9, ou un antigène carcinoembryonnaire.

6. Utilisation d'un réactif dans un procédé de mesure d'un marqueur tumoral par dosage immunologique, selon la revendication 1, le réactif comprenant un liquide de prétraitement contenant un tensioactif anionique ou un acidifiant.
